# EUROPÄISCHE PATENTSCHRIFT

(11) **EP 0 766 985 B1**
(45) Veröffentlichungstag und Bekanntmachung des Hinweises auf die Patenterteilung: **24.01.2001**
(21) Anmeldenummer: 96115819.3
(22) Anmeldetag: 02.10.1996
(51) Int. Cl.: B01D 9/00

(54) **Verfahren und Vorrichtung zur Trennung flüssiger eutektischer Mischungen durch Kristallisation an Kühlflächen**
Process and device for the separation of liquid eutectic mixtures by crystallisation on cooling surfaces
Procédé et dispositif de séparation de mélanges liquides eutectiques par cristallisation aux surfaces de refroidissement

(30) Priorität: 02.10.1995 DE 19536827
(43) Veröffentlichungstag der Anmeldung: 09.04.1997
(73) Patentinhaber: BASF AKTIENGESELLSCHAFT, 67056 Ludwigshafen (DE)
(72) Erfinder: Borho, Klaus, Dr., 67112 Mutterstadt (DE); Heilek, Jörg, 69245 Bammental (DE); Schnabel, Gunter, Dr., 67136 Fussgönheim (DE)
(74) Vertreter: Isenbruck, Günter, Dr.

(56) Entgegenhaltungen:
- EP-A- 0 279 439
- GB-A- 460 834
- US-A- 5 434 316

## Beschreibung

Die Erfindung betrifft ein Verfahren und eine Vorrichtung zur Trennung flüssiger eutektischer Mischungen durch Kristallisation an Kühlflächen, auf die Impfkristalle in Form einer Impfkristallschicht aufgebracht werden, auf denen arteigene Eutektikumsbildner aufwachsen, welche nach Erwärmung der Flächen in flüssiger Form abgezogen werden.

Die Stofftrennung durch Kristallisation ist von besonderem Interesse, wenn Enantiomerenmischungen d.h. Mischungen optisch aktiver Isomeren, getrennt werden sollen. In diesen Fällen weisen andere thermische Trennverfahren (Extraktion, Destillation) keine Selektivität auf; lediglich durch den aufwendigen Einsatz von Hilfsstoffen (chirale Substanzen) kann bei diesen Verfahren eine Trennwirkung erreicht werden.

In der Regel ist es erforderlich, die Stofftrennung mit einer möglichst großen, auf das Wertprodukt bezogenen Ausbeute durchzuführen. Hierin liegt ein wesentliches Problem der Kristallisation als Trennverfahren, da Wertstoff und unerwünschte Nebenkomponenten in den meisten Fällen eutektische Mischungen aufweisen, die ohne besondere Maßnahmen nicht weiter aufzutrennen sind. Demnach treten in diesen Fällen Wertstoff-Ausbeuteverluste entsprechend der eutektischen Zusammensetzungen auf. Im Falle von racemischen Enantiomerenmischungen, bei denen das Racemat gleichzeitig die eutektische Mischung darstellt (racemisches Konglomerat), ist ohne besondere Maßnahmen auch mittels Kristallisation keine Stofftrennung möglich.

Man hat daher bereits die Kristallisation als Trennverfahren eingesetzt, um auch eutektische Zusammensetzungen aufzutrennen. Hierzu ist bekannt, daß eutektische Mischungen getrennt werden können, indem man einzelne Impfkristalle der eutektikumsbildenden Substanzen (im folgenden auch EB = Eutektikumsbildner genannt) in Schmelzen oder Lösungen mit eutektischer Zusammensetzung (im folgenden eutektische Flüssigkeit genannt) vorlegt, die Impfkristalle unter definierten Bedingungen hinsichtlich Übersättigung (innerhalb des metastabilen Bereichs) durch selektives Aufwachsen der EB auf die arteigenen Impfkristalle weiterwachsen läßt und die einzelnen Kristalle nach dem Wachstum durch geeignete Maßnahmen weitgehend sortenrein aus der Schmelze oder Lösung abtrennt.

Bei bekannten Kristallisationsverfahren und -apparaten zur Trennung von eutektischen Mischungen, insbesondere zur Trennung von Enantiomeren werden die EB entweder selektiv in zwei gekoppelten Kristallisatoren (DE-PS 18 07 495, DE-PS 21 09 456) oder in einem gemeinsamen Kristallisator getrennt (US 2,983,757) auskristallisiert. Allen beschriebenen Verfahren/Apparaturen ist gemeinsam, daß die eutektischen Flüssigkeiten mit einzelnen Kristallen der EB angeimpft werden, um selektiv die EB auskristallisieren zu können.

Durch die Vorlage von einzelnen Impfkristallen in die eutektische Flüssigkeit ergibt sich zwangsläufig, daß die EB entweder als disperses Massenkristallisat in einer Suspension (Kristallmaische) erhalten werden oder in speziellen Apparaturen als Einzelkristalle in definierten räumlichen Anordnungen erhalten werden.

Es ist daher eine insbesondere bei Schmelze-Systemen schwierige und technisch aufwendige Abtrennung der suspendierten Kristalle aus der eutektischen Flüssigkeit erforderlich (z.B. durch Zentrifugen, Filter, Schwerkraftabscheider) oder es zwingt die Forderung nach definierter räumlicher Anordnung zu einem aufwendigen Einbringen der einzelnen Impfkristalle in den Kristallisator und zu einem ebenso aufwendigen Sammeln der gewachsenen Kristalle.

Ein weiterer Nachteil der bekannten Verfahren/Apparate ist, daß zum Animpfen der eutektischen Flüssigkeit die Impfkristalle in Form einer Maische oder als Schüttgut gehandhabt werden müssen.

Die vorerwähnten Nachteile führten dazu, daß sich die beschriebenen Verfahren/Apparate in der großtechnischen Anwendung nicht durchsetzen konnten.

EP-A-0 279 439 betrifft ein Verfahren zur Reinigung ausgefrorener Kristallschichten, bei dem die Kristallschichten, nachdem sie aus einer Schmelze oder Lösung auf einer Kühlfläche ausgefroren wurden, unter definierten Bedingungen mit einer Reinigungsflüssigkeit in Kontakt gebracht und damit über ihre gesamte Dicke gereinigt werden, dann von der Reinigungsflüssigkeit abgetrennt und anschließend aufgeschmolzen werden. Hierbei wird nur eine Substanz durch Kristallisation an einer Kühlfläche abgetrennt. GB-A-460 834 offenbart eine Vorrichtung zur Durchführung einer Kristallisation, die zwei Kühlmäntel, bei denen jeweils eine Zuleitung und eine Ableitung für Kühlmittel vorgesehen ist, sowie eine Zuführungsleitung und eine Abführungsleitung aufweist.

Der Erfindung lag daher die Aufgabe zugrunde, ein Verfahren und eine Vorrichtung zur Trennung eutektischer flüssiger Mischungen zu schaffen, die diese Nachteile vermeiden und die in einfacher und zuverlässiger Weise großtechnisch einsetzbar sind.

Erfindungsgemäß wird diese Aufgabe gelöst durch ein Verfahren zur Trennung einer flüssigen eutektischen Mischung wie in Anspruch 1 definiert.

Gemäß einer vorteilhaften Weiterbildung der Erfindung werden die Impfkristallschichten auf den Kühlflächen dadurch gebildet, daß die Kühlflächen mit einer reinen Schmelze, Lösung oder Suspension der eutektikumsbildenden Substanzen in Kontakt gebracht werden und durch Abkühlung die entsprechenden Impfkristallschichten gebildet werden und daß die Kühlflächen danach mit dem zu trennenden Eutektikumsgemisch in Kontakt gebracht werden. Zur Bildung der Impfkristallschichten werden zwei reine Schmelzen, Lösungen oder Supensionen von zwei eutektikumsbildenden Substanzen zur Bildung von Impfkristallschichten auf zwei getrennten Kühlflächen verwendet, die danach mit dem zu trennenden Eutektikumsgemisch in Berührung gebracht werden. Die Begriffe "reine Schmelzen, Lösungen oder Suspensionen" oder "EB-Reinschmelzen" umfassen erfindungsgemäß nicht nur Schmelzen, Lösungen oder Suspensionen von Reinstoffen im herkömmlichen Sinne, sondern alle Schmelzen, Lösungen oder Suspensionen von Mischungen, die in bezug auf die EB reiner sind als das zu trennende, eutektische Gemisch.

Gemäß einer weiteren Ausgestaltung der Erfindung wird beim Aufwachsen der Kristallschichten auf den Kühlflächen die eutektische Mischung im Umlauf gefahren, und nach dem Aufwachsen der Kristallschichten der Umlauf beendet und die eutektische Restflüssigkeit abgeführt.

Danach kann ein Wasch- oder Schwitzschritt durchgeführt werden. Beim Waschen werden die auf den Kühlflächen gewachsenen Kristallschichten mit einer Waschflüssigkeit in Kontakt gebracht und wieder von letzterer getrennt. Dadurch wird die auf den Kristallschichten verbleibende eutektische Restflüssigkeit durch die vorzugsweise reinere Waschflüssigkeit ausgetauscht. Insbesondere bei längerer Verweilzeit der Waschflüssigkeit auf den Kristallschichten erfolgt auch ein diffusiver Austausch von Verunreinigungen zwischen der reineren Waschflüssigkeit und weniger reinen Bereichen der Kristallschichten. Als Waschflüssigkeit werden vorzugsweise frische eutektische Mischung oder EB-Reinschmelze verwendet. Beim Schwitzen wird nach Abführen der eutektischen Restflüssigkeit die Temperatur der Kristallschicht auf einen Wert angehoben, der zwischen den Schmelzpunkttemperaturen der eutektischen Mischung und des EB-Reinstoffes liegt. Dadurch erfolgt ein partielles Abschmelzen von weniger reinen Teilen der Kristallschicht.

Danach werden die Kristallschichten durch Erwärmung verflüssigt und die Flüssigkeiten getrennt abgeführt.

Nach dem getrennten Abführen der eutektikumsbildenden Substanzen werden erneut Kristallschichten von zwei eutektikumsbildenden Substanzen auf zwei getrennten Kühlflächen gebildet, indem jeweils die Kühlfläche mit einer reinen Schmelze, Lösung oder Suspension einer eutektikumsbildenden Substanz in Kontakt gebracht wird und nach Bildung von Impfkristallschichten die eutektische Mischung den Kühlflächen erneut zugeführt wird.

Als Kühlflächen können ebene oder zylindrisch geformte Kühlflächen, z.B. Rohre, eingesetzt werden. Diese Kühlflächen können je mit einem Zulauf und einem Ablauf versehene Teile eines gemeinsamen Wärmetauschers sein.

Das erfindungsgemäße Kristallisationsverfahren arbeitet wie alle bekannten Verfahren/Apparate nach dem grundlegenden Prinzip, im metastabilen Bereich die Eutektikumsbildner (EB) an artgleicher kristalliner Oberfläche aufwachsen zu lassen. Im Gegensatz zu den bereits bekannten Verfahren zeichnet sich das erfindungsgemäße Verfahren jedoch dadurch aus, daß die Trennung der eutektischen Mischung durch Kristallisation an Kühlflächen erfolgt, die mit der eutektischen Flüssigkeit in Kontakt stehen und auf denen zwei EB selektiv als Schichten aufwachsen. Um selektives Kristallschichtwachstum zu erreichen, werden zwei getrennte Kühlflächen vor dem eigentlichen Kristallisiervorgang zunächst mit den reinen Schmelzen, Lösungen oder Suspensionen zweier EB in Kontakt gebracht, danach werden die reinen Schmelzen, Lösungen oder Suspensionen der zwei EB wieder von den Kühlflächen getrennt und die auf den benetzten Kühlflächen verbleibenden dünnen Flüssigkeitsfilme zweier EB werden dann durch Temperaturabsenkung an den Kühlflächen partiell oder vollständig zu Impfkristallschichten erstarrt.

Das Aufbringen einer Impfkristallschicht auf eine Kühlfläche durch Temperaturabsenkung kann auch dadurch erfolgen, daß man den nach Abschmelzen der Kristallschicht aus dem vorangegangenen Zyklus auf der Kühlfläche verbleibenden Flüssigkeitsfilm erstarrt. Sodann werden die mit Impfkristallschichten versehenen Kühlflächen mit eutektischer Flüssigkeit in Kontakt gebracht, und auf den Kühlflächen werden danach durch Temperaturabsenkung zwei EB selektiv zu dickeren Kristallschichten aufwachsen gelassen.

Die im erfindungsgemäßen Verfahren verwendbaren Kühlflächen unterliegen an sich keiner Beschränkung und können beliebiger Form sein. Vorzugsweise werden zylindrisch geformte Kühlflächen, z.B. Rohre, oder ebene Kühlflächen eingesetzt. Hierbei können die Kühlflächen entweder vollständig in eutektische Flüssigkeit eingetaucht sein oder nur von einem Rieselfilm eutektischer Flüssigkeit überströmt werden, z.B. vollständig durchströmtes oder von einem Rieselfilm durchströmtes Rohr.

Das Erstarren eines auf den Kühlflächen verbleibenden dünnen Flüssigkeitsfilms wurde zwar in der DE 17 69 123 und in der DE 37 08 709 zwecks Vorlage kristalliner Oberfläche bereits beschrieben. Dort sind jedoch der Flüssigkeitsfilm und damit auch die resultierende kristalline Phase auf allen Kühlflächen in ihrer Zusammensetzung gleich. Darüber hinaus ist das Vorhandensein einer weiteren kristallinen Phase nicht vorgesehen oder beschrieben. Die Möglichkeit des Vorhandenseins zweier kristalliner Phasen, dadurch erreicht, daß Kühlflächen getrennt und mit in ihrer Zusammensetzung unterschiedlichen Impfkristallschichten versehen werden, wurde nicht erkannt. Entsprechend wurde auch nicht erkannt, daß das Aufbringen von Impfkristallen in Form einer Impfkristallschicht auf einer Kühlfläche zur Trennung von eutektischen Mischungen genutzt werden kann.

Werden durch das erfindungsgemäße Verfahren zur Eutektikumstrennung die EB nicht direkt in ausreichender Reinheit erhalten, können die aus dem erfindungsgemäßen Verfahren kommenden Reinschmelzen der EB mit bekannten Rekristallisationsverfahren weiter hochgereinigt werden.

Der Begriff Eutektikumsbildner umfaßt erfindungsgemäß nicht nur einen einzigen Bestandteil eines eutektischen Gemisches, sondern kann auch Gemische von zwei oder mehr, aber nicht allen eutektikumsbildenden Bestandteilen eines eutektischen Gemisches umfassen.

Das Verfahren eignet sich allgemein zur Trennung aller eutektischen Mischungen, deren einzelne Bestandteile Kristalle bilden können. Besonders vorteilhaft ist das erfindungsgemäße Verfahren zur Trennung von eutektischen Mischungen mit Schmelzpunkten der Mischungen im Bereich von -30°C bis +200°C, vorzugsweise im Bereich zwischen 20 und 150°C. Als Beispiel sei die Trennung einer eutektischen Mischung der Enantiomeren R(+)-2-Phenoxyropionsäure und S(-)-2-Phenoxypropionsäure mit einer Zusammensetzung von R/S = 96/4 und einem Schmelzpunkt von 61°C genannt. Die Kristallisationstemperatur der eutektischen Schmelze beträgt hierbei 61°C, so daß die Kühlflächentemperaturen beim Kristallisieren im Bereich von 61 bis etwa 30°C liegen. Als weiteres Beispiel sei eine eutektische Mischung der Enantiomeren R (+)-2-(4-Chlor-2-methylphenoxy)-propionsäure und S(-)-2-(4-Chlor-2-methylphenoxy)-propionsäure mit einer Zusammensetzung von R/S = 80/20 und einem Schmelzpunkt von 79°C genannt. Die Kristallisationstemperatur der eutektischen Schmelze liegt bei 79°C, weshalb die Kühlflächentemperaturen beim Kristallisieren im Bereich von 79 bis etwa 40°C liegen.

Daneben können mit dem erfindungsgemäßen Verfahren auch eutektische Mischungen von Acrylsäure und ihrer Nebenkomponenten, wie sie bei der Herstellung von Acrylsäure durch Propenoxidation entstehen können, getrennt werden, z.B. Mischungen mit Acrylsäure, Essigsäure, Propionsäure, Benzoesäure, Allylacrylat oder Furan-2-aldehyd als EB. Als Beispiel hierfür sei die eutektische Mischung der Komponenten Acrylsäure und Essigsäure mit einer Zusammensetzung von 55% Acrylsäure/45 % Essigsäure und einem Schmelzpunkt von -27°C genannt.

Das erfindungsgemäße Verfahren zeichnet sich allgemein durch Einfachheit und Betriebssicherheit aus, da kein Massenkristallisat als Feststoff gehandhabt werden muß, also keine Abtrennung auf Filtern oder Zentrifugen und keine Feststofftransportvorgänge erforderlich sind. Speziell als Verfahren zur Trennung eutektischer Mischungen liegt der besondere Vorteil darin, daß die aufwendige Handhabung der Impfkristalle in Form einer Maische oder als Schüttgut entfällt. Stattdessen wird die zur selektiven Kristallisation erforderliche Vorlage kristalliner Oberfläche auf einfachste Weise über das Aufbringen einer Impfkristallschicht auf eine Kühlfläche, bevorzugt durch das Erstarren eines Flüssigkeitsfilms auf der Kühlfläche, realisiert.

Die Erfindung betrifft auch eine Vorrichtung wie in Anspruch 7 definiert, zur Durchführung des beschriebenen Verfahrens.

Vorzugsweise weist der Wärmeübertrager (1) ebene oder zylindrisch geformte Kühlfächen auf, wobei vorzugsweise die Wärmeübertrager (la, 1b) eine Baueinheit (1) bilden.

Vorzugsweise weisen die Kühlflächen der einzelnen Wärmeübertrager (1a, 1b) unterschiedliche Größe oder unterschiedliche Anteile an der Gesamtkühlfläche auf.

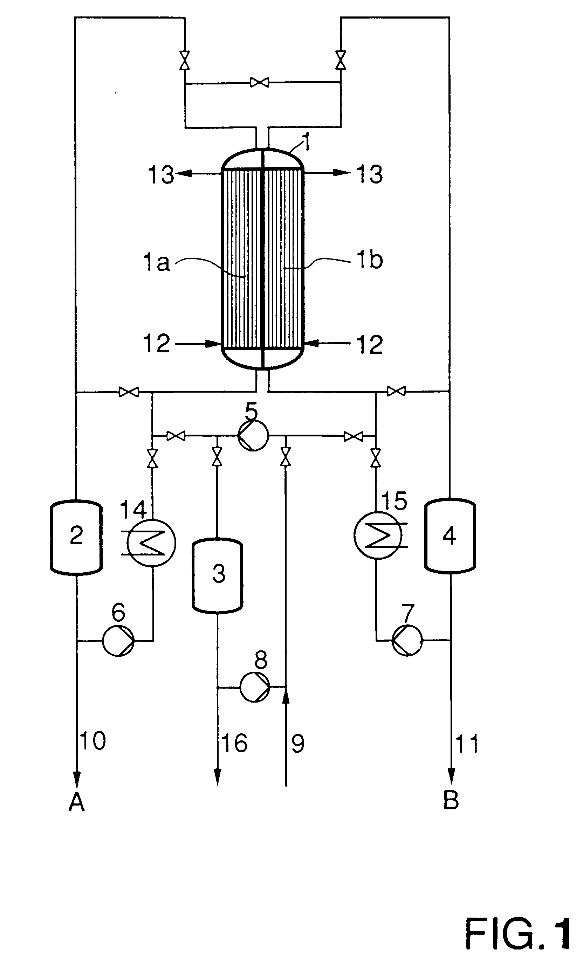

Weitere Einzelheiten und Vorteile der Erfindung können dem in der Figur dargestellten und nachfolgend beschriebenen Ausführungsbeispiel, das eine bevorzugte Ausführungsform der Erfindung darstellt, entnommen werden.

Der in der Figur mit 1 bezeichnete Kristallisator ist baulich in die getrennten Kristallisierzonen la und 1b unterteilt. Im Beispiel ist dies ein 2-zügiger Rohrbündelwärmeübertrager. Drei Behälter 2, 3 und 4 dienen als Auffang- und Vorlagevolumen für die eutektische Flüssigkeit und die Reinschmelzen der EB. Vier Pumpen 5, 6, 7 und 8 dienen der Förderung der im System anfallenden Flüssigkeiten. Mit den Wärmeübertragern 14 und 15 kann man dem System zusätzlich Energie zuführen, sofern dies nicht ausschließlich über den Kristallisator 1 erfolgen soll.

Der Betriebsablauf des Verfahrens ist folgender:

Die Zonen la und 1b des Kristallisators 1 werden zunächst getrennt mit einer der eutektikumsbildenden Substanzen (EB) beaufschlagt. Im vorliegenden Falle wird der Zone la die mit EB A bezeichnete Substanz aus Behälter 2, der Zone 1b die mit EB B bezeichnete Substanz aus Behälter 4 zugeführt. Danach werden die Flüssigkeiten getrennt wieder abgelassen, und zwar A in den Behälter 2, B in den Behälter 4. Daraufhin werden die Kühlflächen abgekühlt, um die auf den Kühlflächen verbleibenden Flüssigkeitsfilme zu Impfkristallschichten zu erstarren. Die Abkühlung der Kühlflächen in den beiden Zonen des als Kristallisator dienenden Wärmeübertragers erfolgt über ein Kühlmittel, das durch die Rohrleitung 12 zugeführt und durch die Rohrleitung 13 abgeführt wird.

Danach wird die zu trennende eutektische Flüssigkeit mit ihren Bestandteilen A und B über die Zuleitung 9 in den Kristallisator 1 eingeleitet und über die Pumpe 5 durch die Rohre der Kristallisierzonen 1a und 1b kontinuierlich im Kreis gefahren. Dabei werden die Kühlflächen in den beiden Zonen des Kristallisators mit Hilfe des über die Leitungen 12 zugeführten und die Leitungen 13 abgeführten Kühlmittels in der Weise abgekühlt, daß die EB in den Kristallisierzonen 1a und 1b selektiv auf den vorhandenen Impfkristallschichten aufwachsen und keine neuen Kristallkeime entstehen.

Nach Erreichen einer gewünschten Schichtdicke wird der Abkühlvorgang abgebrochen, die Pumpe 5 außer Betrieb genommen und die im Kristallisator 1 verbliebene, eutektische Restflüssigkeit in den Behälter 3 abgelassen.

Die in den Kristallisierzonen la und 1b auf den Kühlflächen haftenden Kristallschichten werden durch Anheben der Kühlflächentemperaturen auf Temperaturen oberhalb der Schmelzpunkte der EB abgeschmolzen. Die abfließenden Schmelzen A und B werden getrennt in den Behältern 2 und 4 aufgefangen (A in Behälter 2, B in Behälter 4). Zur Beschleunigung des Abschmelzvorgangs können über die Pumpen 6 und 7 zusätzlich reine Schmelzen der EB aus den Behältern 2 und 4 herausgefahren und durch die jeweiligen Kristallisierzonen la und 1b geleitet werden. Hierbei kann zusätzlich Energie zum Aufschmelzen über die Wärmeträger 14 und 15 dem System zugeführt werden, wenn dies nicht ausschließlich über den Kristallisator 1 erfolgen soll.

Nach Ablassen der EB-Reinschmelzen aus den Kristallisierzonen la und 1b in die entsprechenden Auffangbehälter 2 und 4 werden durch Absenken der Kühlflächentemperaturen in den Kristallisierzonen die jeweils an den Kühlflächen anhaftenden Restfilme der EB-Reinschmelzen ausgefroren und dienen als Impfkristallschichten für den nächsten Kristallisationszyklus.

Zum erneuten Befüllen des Kristallisators wird frische eutektische Flüssigkeit über Leitung 9 zugeführt. Die in Behälter 3 befindliche eutektische Restflüssigkeit kann über Pumpe 8 ebenfalls zum Befüllen des Kristallisators verwendet werden oder sie wird ganz oder teilweise über Leitung 16 aus dem System abgezogen.

Das vorbeschriebene Verfahren wird dann zyklisch wiederholt. Bei ausreichendem Füllgrad in den Behältern 2 und 4 werden die jeweiligen Reinschmelzen A und B der EB über die Leitungen 10 und 11 aus dem System abgezogen.

Sofern die EB-Reinstoffe deutlich unterschiedliche Schmelztemperaturen aufweisen oder die eutektische Mischung in ihrer Zusammensetzung deutlich von einem Verhälntis 1:1 abweicht, kann dies bei der Zoneneinteilung der Kühlflächen berücksichtigt werden. Bei deutlich unterschiedlichen Schmelzpunkten der EB-Reinstoffe (vorzugsweise bei Temperaturdifferenzen > 10°C) kann es sinnvoll sein, die EB-spezifischen Zonen unabhängig voneinander beheizen und kühlen zu können. Dies ist insbesondere beim Ausfrieren der Impfkristallschichten und Abschmelzen der Kristallschichten von Interesse. Bei eutektischen Zusammensetzungen mit deutlichen Abweichungen vom Verhältnis 1:1 (vorzugsweise ab Verhältnissen 60:40 und ungleicher) kann für den anteilmäßig überwiegenden EB ein entsprechend größerer Kühlflächenanteil vorgesehen werden, für den anderen EB ein entsprechend kleinerer Kühlflächenanteil. Unterschiedliche Kühlflächengrößen, Kühlflächenanzahlen und getrennte Temperierungsmöglichkeiten können durch Segmentierung eines gemeinsamen Wärmeübertragers oder durch Verwendung mehrerer, unterschiedlicher Wärmeübertrager geschaffen werden.

## Patentansprüche

1. Verfahren zur Trennung einer flüssigen eutektischen Mischung durch Kristallisation von zwei eutektikumsbildenden Substanzen aus der Mischung an zwei getrennten Kühlflächen, auf die jeweils Impfkristalle einer eutektikumsbildenden Substanz in Form einer Impfkristallschicht aufgebracht sind, und anschließendes Abziehen der zwei eutektikumsbildenden Substanzen in flüssiger Form nach Erwärmung der Kühlfläche.

2. Verfahren nach Anspruch 1, dadurch gekennzeichnet, daß die Impfkristallschichten auf den Kühlflächen dadurch gebildet werden, daß die Kühlflächen mit reiner Schmelze, Lösung oder Suspension der eutektikumsbildenden Substanzen in Kontakt gebracht werden und durch Abkühlung die entsprechenden Impfkristallschichten gebildet werden, und daß die Kühlflächen danach mit dem zu trennenden Eutektikumsgemisch in Berührung gebracht werden.

3. Verfahren nach Anspruch 1 oder 2, dadurch gekennzeichnet, daß beim Aufwachsen der Kristallschichten auf den Kühlflächen die eutektische Mischung im Umlauf gefahren wird, und nach dem Aufwachsen der Kristallschichten der Umlauf beendet und die eutektische Restflüssigkeit abgeführt wird.

4. Verfahren nach einem der Ansprüche 1 bis 3, dadurch gekennzeichnet, daß man nach Verflüssigung der Kristallschichten durch Erwärmung die Flüssigkeiten getrennt abführt.

5. Verfahren nach einem der vorhergehenden Ansprüche, dadurch gekennzeichnet, daß als Kühlflächen ebene oder zylindrisch geformte Flächen eingesetzt werden.

6. Verfahren nach einem der vorhergehenden Ansprüche, dadurch gekennzeichnet, daß ebene oder zylindrische Kühlflächen, bevorzugt Rohre, von einem Flüssigkeits-Rieselfilm überströmt oder vollständig gefüllt mit Flüssigkeit durchströmt werden.

7. Vorrichtung zur Durchführung des Verfahrens nach einem der vorhergehenden Ansprüche, wobei mindestens zwei mit mindestens einer Zuleitung (12) und einer Ableitung (13) für ein Kühlmittel versehene Wärmeübertrager (1a, 1b), eine Zuführungsleitung und eine Abführungsleitung vorgesehen sind, dadurch gekennzeichnet, daß die getrennten Zuführungsleitungen der Wärmeübertrager (1a, 1b) mit einer gemeinsamen Zuführungsleitung (9) verbindbar sind, und die Abführungsleitungen der Wärmeübertrager (1a, 1b) mit je einem getrennten (2, 4) oder mit einem gemeinsamen (3) Auffangbehälter verbindbar sind.

8. Vorrichtung nach Anspruch 7, dadurch gekennzeichnet, daß der Wärmeübertrager (1) ebene oder zylindrisch geformte Kühlflächen aufweist.

9. Vorrichtung nach Anspruch 8, dadurch gekennzeichnet, daß die Wärmeübertrager (la, 1b) eine Baueinheit (1) bilden.

## Claims

1. A process for separating a liquid eutectic mixture by crystallization of two eutectic-forming substances from the mixture on two separate cold surfaces to each of which seed crystals of a eutectic-forming substance are applied in the form of a seed crystal layer, and subsequent removal of the two eutectic-forming substances in liquid form after the cold surface has been heated.

2. A process as claimed in claim 1, wherein the seed crystal layers are formed on the cold surfaces by bringing the cold surfaces into contact with pure melt, solution or suspension of the eutectic-forming substances and the corresponding seed crystal layers are formed by cooling, and the cold surfaces are then brought into contact with the eutectic mixture to be separated.

3. A process as claimed in claim 1 or 2, wherein the eutectic mixture is circulated during growing of the crystal layers on the cold surfaces, and, after the growth of the crystal layers, the circulation is terminated and the residual eutectic liquid is removed.

4. A process as claimed in any of claims 1 to 3, wherein the liquids are removed separately after liquefaction of the crystal layers by heating.

5. A process as claimed in any of the preceding claims, wherein the cold surfaces used are flat or cylindrical surfaces.

6. A process as claimed in any of the preceding claims, wherein a liquid trickle film flows over, or liquid completely fills and flows through, flat or cylindrical cold surfaces, preferably tubes.

7. Apparatus for carrying out a process as claimed in any of the preceding claims, at least two heat exchangers (la, 1b) provided with at least one feed pipe (12) and one discharge pipe (13) for a coolant, a feed pipe and a discharge pipe being provided, wherein the separate feed pipes of the heat exchangers (la, 1b) are capable of being connected to a common feed pipe (9) and the discharge pipes of the heat exchangers (la, 1b) are capable of being connected each to a separate collecting container (2, 4) or to a common collecting container (3).

8. Apparatus as claimed in claim 7, wherein the heat exchanger (1) has flat or cylindrical cold surfaces.

9. Apparatus as claimed in claim 8, wherein the heat exchangers (la, 1b) form a structural unit (1).

## Revendications

1. Procédé de séparation d'un mélange liquide eutectique par cristallisation de deux substances formant un eutectique, à partir du mélange, sur deux surfaces de refroidissement séparées, sur lesquelles sont respectivement déposés des cristaux d'inoculation d'une des substances formant un eutectique sous forme d'une couche de cristaux d'inoculation, puis on extrait sous forme liquide les deux substances formant un eutectique après réchauffement des surfaces de refroidissement.

2. Procédé selon la revendication 1, caractérisé en ce que les couches de cristaux d'inoculation sont formées sur les surfaces de refroidissement par mise en contact des surfaces de refroidissement avec une masse fondue, une solution ou une suspension pures des substances formant un eutectique, et les couches de cristaux d'inoculation correspondantes sont formées par refroidissement, et les surfaces de refroidissement sont ensuite mises en contact avec le mélange eutectique à séparer.

3. Procédé selon la revendication 1 ou 2, caractérisé en ce que, lors de la croissance des couches de cristaux sur les surfaces de refroidissement, le mélange eutectique est mis en circulation, et après croissance des couches de cristaux, la circulation est interrompue et le liquide eutectique résiduel est évacué.

4. Procédé selon l'une quelconque des revendications 1 à 3, caractérisé en ce que l'on évacue les liquides de façon séparée, après fluidification des couches de cristaux par chauffage.

5. Procédé selon l'une quelconque des revendications précédentes, caractérisé en ce que l'on utilise des surfaces planes ou en en forme de cylindre en tant que surfaces de refroidissement.

6. Procédé selon l'une quelconque des revendications précédentes, caractérisé en ce que des surfaces de refroidissement planes ou cylindriques, de préférence des tubes, sont traversées par un film ruisselant de liquide ou en étant entièrement remplies avec du liquide.

7. Dispositif de mise en oeuvre du procédé selon l'une quelconque des revendications précédentes, comprenant au moins deux échangeurs de chaleurs (1a, 1b) comportant au moins une conduite d'entrée (12) et une conduite de sortie (13) pour un réfrigérant ainsi qu'une conduite d'alimentation et une conduite d'évacuation, caractérisé en ce que des conduites d'alimentation séparées des échangeurs de chaleur (1a, 1b) sont reliables par une conduite d'alimentation commune (9), et les conduites d'évacuation des échangeurs de chaleur (la, 1b) sont reliables avec à chaque fois un conteneur de réception séparé (2, 4) ou commun (3).

8. Dispositif selon la revendication 7, caractérisé en ce que l'échangeur de chaleur (1) présente des surfaces de refroidissement planes ou de forme cylindrique.

9. Procédé selon la revendication 8, caractérisé en ce que les échangeurs de chaleur (1a, 1 b) forment une unité structurelle (1).
